# EUROPEAN PATENT SPECIFICATION

(11) **EP 1 289 474 B1**
(45) Date of publication and mention of the grant of the patent: **09.05.2007**
(21) Application number: 01937780.3
(22) Date of filing: 24.05.2001
(51) Int. Cl.: A61K 8/06, B01F 17/00

(54) **LOW EMULSIFIER MULTIPLE EMULSIONS**
MEHRPHASEN EMULSIONEN MIT NIEDRIGEN EMULGIERMITTEL
EMULSIONS MULTIPLES A EMULSIFIANTS FAIBLES

(30) Priority: 26.05.2000 US 580743; 28.02.2001 US 795423
(43) Date of publication of application: 12.03.2003
(73) Proprietor: Color Access, Inc., Melville, New York 11747 (US)
(72) Inventor: MATATHIA, Michelle, Plainview, NY 11803 (US); TADLOCK, Charles, Craig, Islip Terrace, NY 11752 (US)
(74) Representative: Hirsch & Associés
(86) International application number: PCT/US2001/017234
(87) International publication number: WO 2001/091703

(56) References cited:
- EP-A- 0 715 842
- EP-A- 0 779 071
- EP-A- 0 780 112
- EP-A- 0 908 170
- EP-A- 0 970 741
- EP-A- 0 985 402
- WO-A-01/85108

## Description

### Field of the Invention

The present invention relates to cosmetic and pharmaceutical compositions. More specifically, the invention relates to cosmetic and pharmaceutical emulsions.

### Background of the Invention

One of the most common vehicles for cosmetic and pharmaceutical products is the emulsion. Because they are formed by the dispersion of an oil in water, or water in an oil, emulsions provide great versatility in the delivery of different types of active ingredients. A single oil and water formulation can be used to deliver both oil soluble and water soluble active components, thereby giving the formulation a range of potential activity that cannot be matched by a single phase system.

An obvious disadvantage to emulsions is that the materials to be combined are not inherently compatible. The natural tendency of oil and water to separate when mixed must therefore be compensated for by addition of further components to the formulation to aid in keeping the components of the dispersion together. Typically, maintenance of a stable dispersion requires the addition of substantial amounts of emulsion stabilizers and/or emulsifiers. The necessity of addition of these materials not only adds cost to the final product, but also has an effect on the quality of the final product, by affecting the way the emulsion breaks, as well as how it feels on the skin. Use of large quantities of emulsifiers is particularly undesirable, as many consumers perceive these materials as being potentially harsh or irritating to the skin.

The problem in further magnified when the formulation desired is a multiple emulsion, for example, a water-in-oil-in water, or oil-in-water-in-oil. Such emulsions, when feasible, provide a multipurpose product, at least in principle permitting the inclusion of an even greater number of different actives or other cosmetic components, with varied incompatibilities to heat, other components, or one of the desired solvents. It also is a useful vehicle for delayed release of actives on and into the skin, by virtue of the necessity of passing through the multiple phases. However, in practice, despite their clear value, such emulsions are not frequently employed, as the additional phase introduces further problems with stability, and therefore, they frequently require the use of very large quantities of emulsifiers and/or emulsion stabilizers. Further, once a particular system is established, the addition of other materials to the stable emulsion will tend to destabilize it. Therefore, the full potential of the multiple emulsion has not been fully realized. The present invention, however, provides an advance in the preparation of low-emulsifier multiple emulsions.

EP908170 teaches a combination of polymers which is suitable for gelling the outer aqueous phase of a triple emulsion and which makes it possible to obtain a water/oil/water (W/O/W) triple emulsion which is stable even in the presence of acidic active agents.

The combination of polymers comprises at least one emulsifier copolymer composed to a major extent of a monoolefinically unsaturated C3-C6 carboxylic acid monomer or of its anhydride and to a minor extent of an acrylic acid fatty ester monomer, and at least one crosslinked poly(acrylamidomethylpropanesulfonic acid) comprising, distributed randomly, a compound of formula III designated in the specification (col. 2, lines 11-22) and from 0.01 to 10% by weight of crosslinking units originating from at least one monomer having at least two olefin double bonds.

### Summary of the Invention

The present invention relates to a stable water in oil in water emulsion comprising a primary emulsion in an external phase, and comprising a principle water phase and a principle oil phase, the multiple emulsion of an emulsifier having an HLB of 16 to 20 wherein the principle water phase is thickened with an ammonium poly(acryldimethyltauramide-co-vinylformamide, an AMPS/VIFA copolymer, and wherein the emulsifier is present at no more than 1%. Preferably, the principle oil phase is thickened by the addition of an oil miscible polymer having polar moieties. In a preferred embodiment, particularly in the water-in-oil-in water type of emulsion, the viscosity of the two components, i.e., the primary emulsion and the external phase, are adjusted so as to be substantially the same. In such an embodiment, the viscosity of the principle water phase is adjusted by addition of a small amount, preferably less than 2%, of a water-miscible thickener with no emulsifying properties. Multiple emulsions prepared in this way are remarkably stable, and because of the low level of emulsifiers, very gentle to the skin.

### Detailed Description of the Invention

The emulsions of the invention are prepared in much the same way as other multiple emulsions are prepared. Initially, a water-in-oil or an oil-in-water emulsion is prepared according to standard procedure. For a standard emulsion, the water soluble ingredients are combined together in an aqueous vehicle, the oil soluble ingredients are combined in the oil vehicle, and the two phases are combined with a standard homogenizer. In the present case, the water and oil components can be any of the standard components that are ordinarily used for this purpose. The aqueous phase may be any cosmetically acceptable water based material, such as deionized water, or a floral water. The oil phase may be any cosmetically or pharmaceutically acceptable oil, such an oil being defined for the present purpose as any pharmaceutically or cosmetically acceptable material which is substantially insoluble in water. As the oils can perform different functions in the composition, the specific choice is dependent on the purpose for which it is intended. The oils may be volatile or non-volatile, or a mixture of both. For example, suitable volatile oils include, but are not limited to, both cyclic and linear silicones, such as octamethylcyclotetrasiloxane and decamethylcyclopentasiloxane; or straight or branched chain hydrocarbons having from 8-20 carbon atoms, such as decane, dodecane, tridecane, tetradecane, and C8-20 isoparaffins.

Non-volatile oils include, but are not limited to, vegetable oils, such as coconut oil, jojoba oil, corn oil, sunflower oil, palm oil, soybean oil; carboxylic acid esters such as isostearyl neopentanoate, cetyl octanoate, cetyl ricinoleate, octyl palmitate, dioctyl malate, coco-dicaprylate/caprate, decyl isostearate, myristyl myristate; animal oils such as lanolin and lanolin derivatives, tallow, mink oil or cholesterol; glyceryl esters, such as glyceryl stearate, glyceryl dioleate, glyceryl distearate, glyceryl linoleate, glyceryl myristate; non-volatile silicones, such as dimethicone, dimethiconol, dimethicone copolyol, phenyl trimethicone, methicone, simethicone; and nonvolatile hydrocarbons, such as isoparaffins, squalane, or petrolatum.

Although any oil can be used, and mixtures of different types of oils is contemplated, it is particularly preferred that the principle component of the oil phase be a silicone oil, particularly dimethicone, cyclomethicone, or a combination of both. Most preferably, the silicone portion of the emulsion should be about 15-50% of the total water-in-silicone emulsion. Incorporated into the oil phase is an oil-miscible polymer having polar moieties. The polymer provides some level of thickening, and also, because of the presence of the polar groups, assists holding together the water and oil phases, thereby conferring a substantial level of stabilization. Any polymer fitting this description can be used. A particularly preferred polymer for this purpose, especially in a silicone oil base, is a dimethicone copolyol crosspolymer. Material of this type can be purchased from Shin-Etsu Silicones, under the product name KSG21. Another example of a useful oil-phase thickener, which may be more appropriate for a non-silicone oil phase, is lanolin; although not strictly speaking a polymer, it has many of the characteristics of a polymer, with its high molecular weight and thickening properties. It is also oil-miscible, and yet has the requisite polar groups in the presence of fatty alcohols and esters. Therefore, when used in the present context, the term "polymer" will be understood to encompass this type of complex molecule as well. The polymer in absolute terms will be used in an amount suitable to the desired viscosity. Generally speaking, the polymer will be present in an amount greater than zero but no greater than 5%, preferably no greater than about 3%, more preferably no greater than about 2%, most preferably no greater than about 1%, by weight of the total multiple emulsion

To prepare a water-in-silicone(or oil)-in water emulsion, the simple emulsion is added to a water phase which will serve as the external phase of the multiple emulsion. The proportion of emulsion to the water phase can be up to 50:50, but preferably is in the range of about 10-40:90-60 emulsion:water, and most preferably is in the range of about 30-40:70-60. In order to enhance the stability, the external water phase is also thickened with any water-miscible thickener, provided it does not have emulsifying properties. Examples of useful thickeners for use in the external water phase are gums, such as xanthan gum, carbomer, cellulosics, chitosan, starches, and the like. The thickener for the external water phase is an ammonium poly(acryldimethyltauramide-co-vinylformamide), also referred to as AMPS/VIFA copolymer, available commercially from Clariant Corporation, Charlotte, N.C. under the name trade name Aristoflex AVCÒ. The amount of thickener is not crucial, and in this type of emulsion will be used in an amount sufficient to give the desired viscosity.

Also added to the water phase, again in relatively small quantities, is a traditional emulsifier having an HLB in the range of about 16-20. The majority of emulsifiers falling into this category are ethoxylates, most frequently nonionic ethoxylated fatty acids, esters, sorbitan esters, oils and alkylphenols. However, any type of liquid emulsifier meeting the HLB requirement can be used. Examples of other emulsifiers of this type can be found in McCutcheon's, Vol 1: Emulsifiers & Detergents, 2000. Particularly preferred for use in the present invention is Tween 20 (POE (20) sorbitan monolaurate) with an HLB of about 16.7. Unlike more typical multiple emulsions, there is very little of this standard emulsifier needed to hold the emulsion together. Overall, there will ordinarily be no more than 2% total emulsifier of any kind in the multiple emulsion, and preferably no more than 1%, more preferably 0.5% or less (by weight of the multiple emulsion) of a standard ethoxylated emulsifier. The high HLB emulsifier is added to the principle water phase after gelling and just prior to combination with the water-in-oil primary emulsion. The two entities are then combined by static mixing, and mixed to homogeneity.

The foregoing system has been described in terms of a water-in-oil-in-water emulsion.

The emulsions prepared as described above are highly stable. However, additional stability, particularly with the water-in-oil-in water emulsion, can be obtained by matching the viscosities of the primary emulsion and the external phase. As already noted above, the overall viscosity of the product is a matter of choice, depending on the intended final use of the product. However, it is preferred, within that framework, that the viscosities of the emulsion and external phase be matched to within about 10%, viscosity being measured in centipoise by a Brookfield viscometer.

The emulsions of the present invention provide a number of advantages over traditional multiple emulsions. For example, they are prepared with a minimum quantity of emulsifiers and thickeners, the presence of which can alter the mildness and the desired elegant feel of the final product. The system also permits for a greater concentration of the primary emulsion (10-50%) in the multiple emulsion, thereby permitting a broader variety of textures, and a broader appeal to a wide range of consumers. As with other multiple emulsions, these can be used to deliver a number of different types of active materials, partitioned among the various phases of the final product. This can be particularly important in a system in which there are several actives that may not be compatible together, or that may not exhibit optimum activity in the same environment. The multiple emulsions can also be used as a novel delivery system for pigment, in which the pigment is incorporated into the internal phase of the emulsion, and the color developed after rubbing on the skin. The emulsions can essentially be used for any type of application in which a standard emulsion is routinely used, for example, skin care products, pharmaceutical or veterinary drug delivery, sunscreens/self-tanners, rinse-off hair conditions, and liquid makeups.

The invention will be further illustrated by the following non-limiting examples.

### EXAMPLES

### Example 1.

### A. Preparation of primary emulsion for a triple emulsion foundation

| **Material** | **Weight %** |
|---|---|
| Phase 1 | |
| Cyclomethicone/dimethicone | 5.00 |
| Phenyl trimethicone | 5.00 |
| Dimethicone/dimethicone copolyol | |
| Crosspolymer(75:25) | 7.00 |
| Cyclomethicone | 1.00 |
| Dimethicone | 8.00 |
| Pigment | 5.00 |
| Elefac I-205 | 3.00 |
| | |

| Phase 2 | |
|---|---|
| Xanthan gum | 0.20 |
| Butylene glycol | 5.00 |
| Distilled water | 59.80 |
| Sodium chloride | 1.00 |

Phase 1 materials are combined with sweep mixing in a primary vessel. The xanthan gum and butylene glycol are combined in an auxiliary vessel and mixed under propeller mixing until homogeneous. The remaining Phase 2 materials are added to the auxiliary vessel and heated to 40°C, mixing with propeller. Phase 2 materials are added to Phase 1 materials, and then homomixed to desired viscosity.

### B. Preparation of triple emulsion

| (i) external water phase | | |
|---|---|---|
| | **Material** | **Weight %*** |
| | Distilled water | 49.70 |
| | Glycerine/glyceryl polyacrylate | 1.00 |
| | Sodium hyaluronate (2% solution) | 10.00 |
| | Dimethicone copolyol | 0.50 |
| | Glycereth-26 | 2.00 |
| | 1,3 butylene glycol | 5.00 |
| | Tween 20 | 0.30 |
| | | |

| (ii) thickener | | |
|---|---|---|
| | AMPS/VIFA copolymer** | 1.50 |
| | | |

| (iii)primary emulsion | | |
|---|---|---|
| | composition of Example 1A | 30.00 |

| | | |
|---|---|---|
| *of total multiple emulsion ** ammonium poly(acryldimethyltauramide-co-vinylformamide)Aristoflex AVCÒ, Clariant Corporation | | |

The materials of the external water phase are combined and heated to 45°C with propeller mixing. With static mixing, the thickener is added to the water phase and mixed until a clear gel is formed. The primary emulsion is added to the previously combined materials under continuous static mixing until mixed to completion.

### Example 2.

### Preparation of a quadruple emulsion (outside claimed scope)

### A. A water-in-oil primary emulsion is prepared as follows:

| Material | Weight % |
|---|---|
| Phase I | |
| Cyclomethicone/dimethicone | 5.00 |
| Phenyltrimethicone | 5.00 |
| Dimethicone/copolyol crosspolymer | 7.00 |
| Cyclomethicone | 1.00 |
| Dimethicone | 8.00 |
| | |

| Phase II | |
|---|---|
| Xanthan gum | 0.20 |
| Deionized water | 64.30 |
| Sodium chloride | 1.00 |
| Butylene glycol | 5.00 |
| Parabens | 0.50 |

The oil phase ingredients are combined together, and the water phase ingredients are combined together. The water phase is then slowly added to the oil phase, and homogenized until uniform.

### B. Water-thin, low emulsifier emulsion serving as the external "water" phase is prepared as follows:

| Material | Weight % |
|---|---|
| | |
| Phase I | |
| deionized water | 32.50 |
| Arlatone Versaflex High Performance Emulsion Stabilizer* | 1.00 |
| | |

| Phase II | |
|---|---|
| Deionized water | 32.05 |
| Methyl paraben | 0.20 |
| Butylene glycol | 3.00 |
| Phenoxyethanol | 0.40 |
| | |

| Phase III | |
|---|---|
| Behenyl alcohol | 0.75 |
| Pentaerythrityl tetraethylhexanoate | 30.00 |
| Beta-carotene | 0.10 |

| | |
|---|---|
| *Uniqema | |

In Phase I, the emulsifier is added to water at 80°C. Phase II ingredients are added to Phase I at 80°C. Phase III ingredients are combined and then homomixed with Phase I and II ingredients at greater than 10,000 rpm for 5 minutes. The combined components are then passed through a microfluidizer at 16,000 psi three times to achieve a water-thin emulsion.

| Material | Weight % |
|---|---|
| | |
| Polysorbate 20 | 0.20 |
| Carbopol | 1.00 |
| O/W emulsion from B. | 78.80 |
| W/O emulsion from A. | 20.00 |

### C. Quadruple emulsion

The O/W emulsion is combined with the Carbopol using static mixing. Polysorbate 20 is then added. The W/O emulsion is slowly added to the O/W phase utilizing static mixing. When the addition is complete, the mixing is continued for about 5 minutes until the multiple emulsion is uniform.

## Claims

1. A stable water in oil in water emulsion comprising a primary emulsion in an external phase, and comprising a principle water phase and a principle oil phase, the multiple emulsion containing an emulsifier having an HLB of 16 to 20 wherein the principle water phase is thickened with an ammonium poly(acryldimethyltauramide-co-vinylformamide, an AMPS/VIFA copolymer, and wherein the emulsifier is present at no more than 1%.

2. The emulsion of claim 1 in which the principle oil phase is thickened with an oil-miscible polymer having polar moieties.

3. The emulsion of claim 1 or 2 in which the principle oil phase comprises primarily silicone oil.

4. The emulsion of claim 2 or 3 in which the polymer is a dimethicone copolyol crosspolymer.

5. The emulsion of any one of claims 1 to 4 in which the principle water phase is thickened by a water-miscible thickener.

6. The emulsion of any one of claims 1 to 5 in which the water miscible thickener is selected from the group consisting of gums, carbomer, cellulosics, chitosan, and starches.

7. The emulsion of any one of claims 1 to 6 in which the viscosity of the primary emulsion and the viscosity of the external phase are matched to within 10%.

## Patentansprüche

1. Stabile Wasser-in-Öl-in-Wasser-Emulsion, die eine primäre Emulsion in einer äußeren Phase umfasst und eine Hauptwasserphase und eine Hauptölphase umfasst, wobei die multiple Emulsion einen Emulgator mit einem HLB-Wert von 16 bis 20 aufweist, wobei die Hauptwasserphase mit einem Ammonium-Acryldimethyltauramid-Vinylformamid-Kopolymer, einem AMPS/VIFA-Kopolymer, verdickt ist, und wobei der Emulgator nicht mehr als 1 % ausmacht.

2. Emulsion nach Anspruch 1, wobei die Hauptölphase mit einem in Öl mischbaren Polymer mit polaren Komponenten verdickt ist.

3. Emulsion nach Anspruch 1 oder 2, wobei die Hauptölphase hauptsächlich ein Silikonöl umfasst.

4. Emulsion nach Anspruch 2 oder 3, wobei das Polymer ein Dimethicon-Copolyol-Kreuzpolymer ist.

5. Emulsion nach einem der Ansprüche 1 bis 4, wobei die Hauptwasserphase mit einem in Wasser mischbaren Verdickungsmittel verdickt ist.

6. Emulsion nach einem der Ansprüche 1 bis 5, wobei das in Wasser mischbare Verdickungsmittel aus der Gruppe ausgewählt ist, die aus Gummi, Carbomer, Cellulosederivaten, Chitosan und Stärken besteht.

7. Emulsion nach einem der Ansprüche 1 bis 6, wobei die Viskosität der primären Emulsion und die Viskosität der äußeren Phase innerhalb von 10% aneinander angeglichen sind.

## Revendications

1. Une émulsion eau-dans-huile-dans-eau stable comprenant une émulsion primaire dans une phase externe, et comprenant une phase aqueuse principale et une phase huileuse principale, cette émulsion multiple contenant un agent émulsifiant présentant une HLB comprise entre 16 et 20, dans laquelle la phase aqueuse principale est épaissie par un poly(acryldiméthyltauramide-co-vinylformamide, un copolymère AMPS/VIFA et dans laquelle l'agent émulsifiant est présent en une proportion non supérieure à 1 %.

2. L'émulsion selon la revendication 1, dans laquelle la phase huileuse principale est épaissie par un polymère miscible à l'huile comprenant des parties polaires.

3. L'émulsion selon la revendication 1 ou 2, dans laquelle la phase huileuse principale comprend primairement une huile de silicone.

4. L'émulsion selon la revendication 2 ou 3, dans laquelle le polymère est un polymère réticulé à base de diméthicone copolyol.

5. L'émulsion selon une quelconque des revendications 1 to 4, dans laquelle la phase aqueuse principale est épaissie par un agent épaississant miscible à l'eau.

6. L'émulsion selon une quelconque des revendications 1 to 5, dans laquelle l'agent épaississant miscible à l'eau est choisi dans le groupe comprenant gommes, carbomères, matières cellulosiques, chitosan et amidons.

7. L'émulsion selon une quelconque des revendications 1 to 6, dans laquelle la viscosité de l'émulsion primaire et la viscosité de la phase externe sont équivalentes à 10% près.
